# EUROPEAN PATENT SPECIFICATION

(11) **EP 2 550 957 B1**
(45) Date of publication and mention of the grant of the patent: **14.02.2018**
(21) Application number: 11194792.5
(22) Date of filing: 21.12.2011
(51) Int. Cl.: A61K 9/14, A61K 31/155, A61K 9/00

(54) **Effervescent formulations of vildagliptin**
Brauseformulierungen von Vildagliptin
Formulations effervescentes de vildagliptine

(30) Priority: 21.12.2010 TR 201010683; 29.07.2011 TR 201107481
(43) Date of publication of application: 30.01.2013
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Yelken, Gülay, 34398 Istanbul (TR); Saydam, Mehtap, 34398 Istanbul (TR)
(74) Representative: Sevinç, Erkan

(56) References cited:
- WO-A1-2011/064352
- DATABASE WPI Week 200873 Thomson Scientific, London, GB; AN 2008-M35632 XP002680337, & CN 101 234 105 A (BEIJING RUNDEKANG MEDICINE TECHNOLOGY CO) 6 August 2008 (2008-08-06)
- DATABASE WPI Week 200872 Thomson Scientific, London, GB; AN 2008-M15385 XP002680338, & CN 101 181 264 A (BEIJING RUNDEKANG MEDICAL TECHNOLOGY CO) 21 May 2008 (2008-05-21)

## Description

### Field of Invention

The present invention relates to a formulation comprising vildagliptin or a pharmaceutically acceptable salt thereof. The present invention particularly relates to an effervescent powder formulation of vildagliptin, which is stable and provides the desired features.

### Background of Invention

Vildagliptin is a dipeptidyl dipeptidase-IV (DPP-IV) inhibitor developed for use in the treatment of type 2 diabetes (non-insulin dependent diabetes). Vildagliptin inhibits the degradation of the dipeptidyl dipeptidase-IV enzyme, thereby inhibiting the effects of incretin hormones, glucagon-like peptide-1 (GLP-1), and of glucose-dependent insulinotropic peptide (GIP). The chemical designation of vildagliptin is (S)-{[(3-hydroxyadamantan-1-yl)amino]acetyl}pyrrolidine-2-carbonitrile, with the chemical structure illustrated below in Formula 1.

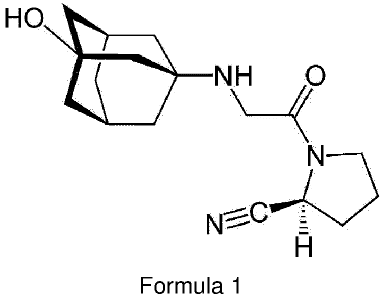

Vildagliptin is soluble in water and in organic polar solvents.

Vildagliptin is marketed under the trademark Galvus® in 50 mg dosage forms. It is used against diabetes mellitus, but particularly in treating type 2 diabetes.

There are various patents in the patent literature in relation to vildagliptin.

The patent application WO0034241 discloses vildagliptin or an acid addition salt thereof, as well as their use in diabetes mellitus and obesity.

The patent application WO2006078593 claims a direct-compression formulation of a DPP-IV inhibitor compound, and preferably of vildagliptin or an acid addition salt thereof.

The patent application WO2006135723 discloses a formulation, comprising vildagliptin as an active agent, as well as hydroxypropyl methyl cellulose, microcrystalline cellulose, and magnesium stearate.

The solubility and dissolution rate of vildagliptin directly influence its bioavailability. For this reason, it is quite important to increase the solubility and dissolution rate of vildagliptin.

Another problem in relation to vildagliptin is stability, which emerges under the influence of ambient and physical conditions, as is the case with many other active agents. Vildagliptin is an active agent that is highly-susceptible to air and humidity. When vildagliptin is exposed to air and humidity, it degrades structurally and develops chemical behavioral changes. The stability of developed vildagliptin products is not at a desired level and the shelf life thereof is shortened. In addition, vildagliptin is reactive against the excipients employed in developing formulations containing the same. This, in turn, causes impurities to occur in the formulation and leads to the inclusion of undesired components into the formulation.

The clarity of a solution, particularly in terms of those agents which are dispersed in water, is a psychologically favorable criterion concerning the use of a medicament, and is thus preferable.

A further problem encountered while developing formulations comprising vildagliptin is flowability. Problems encountered in the flowability of vildagliptin make its production difficult.

Particularly, ease of administration makes the formulations of vildagliptin preferable. Based on the drawbacks and needs mentioned above, a novelty is necessitated in the art of formulations of vildagliptin showing antidiabetic activity.

### Object and Brief Description of Invention

The present invention provides an easily-administrable effervescent vildagliptin composition, eliminating all aforesaid problems and bringing additional advantages to the relevant prior art.

Accordingly, the main object of the present invention is to obtain a stable composition with antidiabetic activity.

Another object of the present invention is to provide a formulation, having a desired level of solubility and dissolution rate, and therefore a desired level of bioavailability, with an effervescent vildagliptin-containing composition.

Another object of the present invention is to obtain a composition which provides a clear dispersion in a fluid by means of preferred excipients.

Another object of the present invention is to develop a composition not leading to flowability-related problems during production.

Accordingly, an effervescent formulation is developed which is dispersed in water for oral administration, in order to carry out any objects, referred to above and to emerge from the following detailed description.

In a preferred embodiment of the present invention, said novelty is realized with particles comprising vildagliptin or a pharmaceutically acceptable salt of vildagliptin, an acid, and a carbonate, as well as an air- and humidity-barrier package into which said particles are filled.

In a preferred embodiment of the present invention, said carbonate is a carbonate or bicarbonate salt of alkali metals.

In a preferred embodiment of the present invention, said carbonate is selected from sodium bicarbonate, sodium bicarbonate anhydrate, sodium hydrogen carbonate, calcium carbonate, sodium glycine carbonate, or from properly-proportioned mixtures thereof.

In a preferred embodiment of the present invention, said acid is selected from sodium citrate, citric acid, tartaric acid, fumaric acid, and malic acid, or from properly-proportioned mixtures thereof.

A preferred embodiment of the present invention comprises at least one or more excipients.

In a preferred embodiment of the present invention, said excipient comprises at least one or a properly-proportioned mixture of diluents, disintegrants, glidants, lubricants, flavoring agents, sweeteners.

In a preferred embodiment of the present invention, the mean particle size (d50) of the vildagliptin composition is in the range of 250 to 600 µm.

In a preferred embodiment of the present invention, said diluent comprises lactose, microcrystalline cellulose, sugar or a properly-proportioned mixture thereof.

In a preferred embodiment of the present invention, said disintegrant comprises at least one or a properly-proportioned mixture of polyvinylpyrrolidone and sodium starch glycolate.

In a preferred embodiment of the present invention, said glidant is colloidal silicon dioxide.

In a preferred embodiment of the present invention, said lubricant preferably comprises at least one or a properly-proportioned mixture of polyethylene glycol and magnesium stearate.

In a preferred embodiment of the present invention, said sweetener comprises at least one or a properly-proportioned mixture of aspartam, sucralose, saccharine, glucose, lactose, fructose and other sugars, and mannitol, sorbitol, xylitol, erythritol, and other sugar alcohols.

In a preferred embodiment according to the present invention, said flavoring agent comprises at least one or a properly-proportioned mixture of menthol, peppermint, cinnamon, chocolate, vanillin, and fruit extracts such as cherry, orange, strawberry, grape.

In another preferred embodiment of the present invention, said pharmaceutical composition comprises the following ingredients only:
a. vildagliptin or a pharmaceutically acceptable salt thereof at 3.5 to 90% by weight,
b. lactose or microcrystalline cellulose, or a properly-proportioned mixture thereof at 5 to 90% by weight,
c. polyvinylpyrrolidone at 1 to 30% by weight,
d. alkali metal carbonate salt at 5 to 60% by weight,
e. tartaric acid or fumaric acid, or a properly-proportioned mixture thereof at 5 to 60% by weight,
f. sweetener at 0.1 to 2% by weight,
g. flavoring agent at 0.1 to 5% by weight.

In another preferred embodiment of the present invention, said pharmaceutical composition comprises the following ingredients only:
a. vildagliptin or a pharmaceutically acceptable salt thereof at 3.5 to 90% by weight,
b. lactose or microcrystalline cellulose, or a properly-proportioned mixture thereof at 5 to 90% by weight,
c. polyvinylpyrrolidone at 1 to 30% by weight,
d. carbonate or bicarbonate salts of alkali metals at 5 to 60% by weight,
e. citric acid or citric acid anhydrate or sodium citrate anhydrate, or a properly-proportioned mixture thereof at 5 to 60% by weight,
f. sweetener at 0.1 to 2% by weight,
g. flavoring agent at 0.1 to 5% by weight.

Another embodiment of the present invention provides a method for preparing said pharmaceutical composition, comprising the steps of
a. mixing vildagliptin or a pharmaceutically acceptable salt thereof with a disintegrant, bicarbonate, acid and lactose or microcrystalline cellulose,
b. adding a sweetener and flavoring agent into the powder mixture produced above and mixing the resulting mixture,
c. adding a glidant and lubricant into the mixture obtained above and mixing the resulting mixture, and
d. filling the resulting powder mixture into packages in a sachet filling machine.

In another preferred embodiment of the present invention, said formulation is in the form of a sachet or tablet.

### Detailed Description of Invention

### Example 1

| **Ingredients** | **% amount (mg)** |
|---|---|
| vildagliptin | %3.5 - 90 |
| polyethylene glycol | %0.5 - 10 |
| polyvinylpyrrolidone | %0.1 - 30 |
| tartaric acid | %5 - 60 |
| sodium bicarbonate or calcium carbonate or a mixture thereof | %5 - 60 |
| sweetener | %0.1 - 2 |
| flavoring agent | %0.1 - 5 |

Vildagliptin or a pharmaceutically acceptable salt thereof is mixed with polyvinylpyrrolidone, sodium bicarbonate or calcium carbonate or a mixture thereof, and tartaric acid. Into the powder mixture obtained are added polyethylene glycol, sweetener, and the flavoring agent and the resulting mixture is mixed again. The mixture obtained is filled into alu - alu packages.

### Example 2

| **Ingredients** | % amount (mg) |
|---|---|
| vildagliptin | %3.5 - 90 |
| lactose or microcrystalline cellulose, or a properly-proportioned mixture thereof | %5 - 90 |
| sugar | %5 - 90 |
| malic acid | %5 - 60 |
| sodium bicarbonate anhydrate or sodium carbonate, or a properly-proportioned mixture thereof | %5 - 60 |
| sucralose | %0.1 - 2 |
| flavoring agent | %0.1 - 5 |

Vildagliptin or a pharmaceutically acceptable salt of vildagliptin is mixed with lactose or microcrystalline cellulose or a properly-proportioned mixture thereof, sodium bicarbonate anhydrate or sodium carbonate or a properly-proportioned mixture thereof, malic acid, sugar, and lactose or microcrystalline cellulose. Into the powder mixture formed are added the sweetener and flavoring agents, and the resulting mixture is mixed again. The mixture obtained is filled into alu - alu packages.

These packages allow to block the contact of the product with air at the desired extent, and to achieve the desired shelf life. The mean particle size of the vildagliptin composition is in the range of 250 to 600 µm, thereby the solubility and the dissolution rate of the formulation and thus its bioavailability are enhanced. Additionally, when the formulation is dispersed in a fluid, the resulting solution shows a clear appearance, and gives the desired features in terms of patient compliance.

With the invention realized, a sachet formulation of vildagliptin can be obtained which has surprisingly good solubility and dissolution rates, and thus a high bioavailability. It is an effervescent formulation administered orally following its dispersion in water, and comprises particles containing vildagliptin or a pharmaceutically acceptable salt of vildagliptin, an acid, and a carbonate, as well as an air- and oxygen-barrier package into which said particles are placed. The dissolution rate and the solubility of vildagliptin is increased with the effect of the effervescent form obtained with the bicarbonates and acids employed.

The pharmaceutical compositions according to the present invention may also comprise one or more pharmaceutically acceptable excipient(s). Such pharmaceutically acceptable excipients include, but are not restricted to fillers, glidants, lubricants, disintegrants, surface active agents etc. and the mixtures thereof.

The present invention is used for preventing or treating diabetes mellitus in mammalians, and particularly in humans.

In this context, the term composition may both correspond to formulations, and to a combined meaning of the formulation and the package or blister in which the formulation is stored.

In this context, the term particle corresponds to a powder, granule, or a pellet.

It is further possible to use the following additional excipients in this formulation.

Lubricants, but are not restricted to at least one or a mixture of sodium stearyl fumarate, polyethylene glycol, stearic acid, metal stearates, boric acid, sodium chloride benzoate and acetate, sodium or magnesium lauryl sulfate, etc..

Preservatives, but are not restricted to at least one or a mixture of methylparaben and propylparaben and salts thereof (e.g. sodium or potassium salts), sodium benzoate, citric acid, benzoic acid, butylated hydroxytoluene and butylated hydroxyanisole.

Surface active agents, but are not restricted to at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate.

The present invention is hereby disclosed by referring to exemplary embodiments hereinabove. Whilst these exemplary embodiments does not restrict the object of the present invention, the latter must be assessed under the light of the foregoing detailed description.

## Claims

1. An effervescent composition, administered orally following its dispersion in water, **characterized by** comprising
particles containing vildagliptin or a pharmaceutically acceptable salt of vildagliptin, an acid, and a carbonate, and
an air- and humidity-barrier package into which said particles are filled.

2. The effervescent pharmaceutical composition according to Claim 1, wherein said carbonate is a carbonate or bicarbonate salt of alkali metals.

3. The pharmaceutical composition according to any of the preceding claims, wherein said carbonate is selected from sodium carbonate, sodium bicarbonate, sodium bicarbonate anhydrate, sodium hydrogen carbonate, calcium carbonate, sodium glycine carbonate, or from properly-proportioned mixtures thereof.

4. The pharmaceutical composition according to any of the preceding claims, wherein said acid is selected from sodium citrate, citric acid, tartaric acid, fumaric acid, and malic acid, or from properly-proportioned mixtures thereof.

5. The pharmaceutical composition according to any of the preceding claims, further comprising at least one or more excipient(s).

6. The pharmaceutical composition according to any of the preceding claims, wherein said excipient comprises at least one or a properly-proportioned mixture of diluents, disintegrants, glidants, lubricants, flavoring agents, and sweeteners.

7. The pharmaceutical composition according to any of the preceding claims, wherein the mean particle size (d50) of said composition is in the range of 250 to 600 µm.

8. The pharmaceutical composition according to any of the preceding claims, wherein said diluent is lactose, microcrystalline cellulose, sugar, or a properly-proportioned mixture thereof.

9. The pharmaceutical composition according to any of the preceding claims, wherein said disintegrant is at least one or a properly-proportioned mixture of polyvinylpyrrolidone and sodium starch glycolate.

10. The pharmaceutical composition according to any of the preceding claims, wherein said glidant is colloidal silicon dioxide.

11. The pharmaceutical composition according to any of the preceding claims, wherein said lubricant preferably comprises at least one or a properly-proportioned mixture of polyethylene glycol and magnesium stearate.

12. The pharmaceutical composition according to any of the preceding claims, wherein said sweeteners comprise at least one or a properly-proportioned mixture of aspartam, sucralose, saccharine; glucose, lactose, fructose and other sugars, and mannitol, sorbitol, xylitol, erythritol, and other sugar alcohols.

13. The pharmaceutical composition according to any of the preceding claims, wherein said flavoring agents comprise at least one or a properly-proportioned mixture of menthol, peppermint, cinnamon, chocolate, vanillin, and fruit extracts such as cherry, orange, strawberry, grape.

14. The pharmaceutical composition according to any of the preceding claims, comprising the following ingredients only:
a. vildagliptin or a pharmaceutically acceptable salt thereof at 3.5 to 90% by weight,
b. lactose or microcrystalline cellulose, or a properly-proportioned mixture thereof at 5 to 90% by weight,
c. polyvinylpyrrolidone at 1 to 30% by weight,
d. carbonate or bicarbonate salts of alkali metals at 5 to 60% by weight,
e. tartaric acid or malic acid, or a properly-proportioned mixture thereof at 5 to 60% by weight,
f. sweetener at 0.1 to 2% by weight,
g. flavoring agent at 0.1 to 5% by weight.

15. The pharmaceutical composition according to any of the preceding claims, comprising the following ingredients only:
a. vildagliptin or a pharmaceutically acceptable salt thereof at 3.5 to 90% by weight,
b. lactose or microcrystalline cellulose, or a properly-proportioned mixture thereof at 5 to 90% by weight,
c. polyvinylpyrrolidone at 1 to 30% by weight,
d. carbonate or bicarbonate salts of alkali metals at 5 to 60% by weight,
e. citric acid or citric acid anhydrate or sodium citrate anhydrate, or a properly-proportioned mixture thereof at 5 to 60% by weight,
f. sweetener at 0.1 to 2% by weight,
g. flavoring agent at 0.1 to 5% by weight.

16. A method for preparing a pharmaceutical composition according to any of the preceding claims, comprising the steps of
a. mixing vildagliptin or a pharmaceutically acceptable salt thereof with a disintegrant, bicarbonate, acid and lactose or microcrystalline cellulose,
b. adding a sweetener and flavoring agent into the powder mixture produced above and mixing the resulting mixture,
c. adding a glidant and lubricant into the mixture obtained above and mixing the resulting mixture, and filling the resulting powder mixture into packages in a sachet filling machine.

17. The pharmaceutical composition according to any of the preceding claims for preventing or treating diabetes mellitus in mammalians and particularly in humans.

18. The pharmaceutical composition according to any of the preceding claims, wherein said composition is in the form of sachet.

## Patentansprüche

1. Brausezusammensetzung, oral verabreicht nach ihrer Dispergierung in Wasser, **gekennzeichnet durch** Umfassen
von Partikeln, enthaltend Vildagliptin oder ein pharmazeutisch verträgliches Salz von Vildagliptin, eine Säure und ein Carbonat, und
einer Verpackung mit Barriere gegen Luft und Feuchtigkeit, in welche die Partikel abgefüllt werden.

2. Pharmazeutische Brausezusammensetzung nach Anspruch 1, wobei das Carbonat ein Carbonat- oder Hydrogencarbonatsalz von Alkalimetallen ist.

3. Pharmazeutische Zusammensetzung nach einem der vorangehenden Ansprüche, wobei das Carbonat ausgewählt ist aus Natriumcarbonat, Natriumhydrogencarbonat, Natriumhydrogencarbonatanhydrat, Natriumhydrogencarbonat, Calciumcarbonat, Natriumglycincarbonat, oder aus geeignet proportionierten Gemischen davon.

4. Pharmazeutische Zusammensetzung nach einem der vorangehenden Ansprüche, wobei die Säure ausgewählt ist aus Natriumcitrat, Citronensäure, Weinsäure, Fumarsäure und Äpfelsäure, oder aus geeignet proportionierten Gemischen davon.

5. Pharmazeutische Zusammensetzung nach einem der vorangehenden Ansprüche, weiterhin umfassend mindestens einen oder mehrere Exzipienten.

6. Pharmazeutische Zusammensetzung nach einem der vorangehenden Ansprüche, wobei der Exzipient mindestens eines oder ein geeignet proportioniertes Gemisch von Verdünnungsmitteln, Sprengmitteln, Fließregulierungsmitteln, Gleitmitteln, Aromastoffen und Süßungsmitteln umfasst.

7. Pharmazeutische Zusammensetzung nach einem der vorangehenden Ansprüche, wobei die mittlere Teilchengröße (d₅₀) der Zusammensetzung im Bereich von 250 bis 600 µm liegt.

8. Pharmazeutische Zusammensetzung nach einem der vorangehenden Ansprüche, wobei das Verdünnungsmittel Lactose, mikrokristalline Cellulose, Zucker, oder ein geeignet proportioniertes Gemisch davon ist.

9. Pharmazeutische Zusammensetzung nach einem der vorangehenden Ansprüche, wobei das Sprengmittel mindestens eines oder ein geeignet proportioniertes Gemisch von Polyvinylpyrrolidon und Natriumstärkeglykolat ist.

10. Pharmazeutische Zusammensetzung nach einem der vorangehenden Ansprüche, wobei das Fließregulierungsmittel kolloidales Siliciumdioxid ist.

11. Pharmazeutische Zusammensetzung nach einem der vorangehenden Ansprüche, wobei das Gleitmittel vorzugsweise mindestens eines oder ein geeignet proportioniertes Gemisch von Polyethylenglykol und Magnesiumstearat umfasst.

12. Pharmazeutische Zusammensetzung nach einem der vorangehenden Ansprüche, wobei die Süßungsmittel mindestens eines oder ein geeignet proportioniertes Gemisch von Aspartam, Sucralose, Saccharin; Glucose, Lactose, Fructose und anderen Zuckern, und Mannitol, Sorbitol, Xylitol, Erythritol und anderen Zuckeralkoholen umfassen.

13. Pharmazeutische Zusammensetzung nach einem der vorangehenden Ansprüche, wobei die Aromastoffe mindestens eines oder ein geeignet proportioniertes Gemisch von Menthol, Pfefferminze, Zimt, Schokolade, Vanillin, und Fruchtextrakten wie Kirsche, Orange, Erdbeere, Weintraube umfassen.

14. Pharmazeutische Zusammensetzung nach einem der vorangehenden Ansprüche, umfassend nur die folgenden Bestandteile:
a. Vildagliptin oder ein pharmazeutisch verträgliches Salz davon zu 3,5 bis 90 Gew.-%,
b. Lactose oder mikrokristalline Cellulose, oder ein geeignet proportioniertes Gemisch davon zu 5 bis 90 Gew.-%,
c. Polyvinylpyrrolidon zu 1 bis 30 Gew.-%,
d. Carbonat- oder Hydrogencarbonatsalze von Alkalimetallen zu 5 bis 60 Gew.-%,
e. Weinsäure oder Äpfelsäure, oder ein geeignet proportioniertes Gemisch davon zu 5 bis 60 Gew.-%,
f. Süßungsmittel zu 0,1 bis 2 Gew.-%,
g. Aromastoff zu 0,1 bis 5 Gew.-%.

15. Pharmazeutische Zusammensetzung nach einem der vorangehenden Ansprüche, umfassend nur die folgenden Bestandteile:
a. Vildagliptin oder ein pharmazeutisch verträgliches Salz davon zu 3,5 bis 90 Gew.-%,
b. Lactose oder mikrokristalline Cellulose, oder ein geeignet proportioniertes Gemisch davon zu 5 bis 90 Gew.-%,
c. Polyvinylpyrrolidon zu 1 bis 30 Gew.-%,
d. Carbonat- oder Hydrogencarbonatsalze von Alkalimetallen zu 5 bis 60 Gew.-%,
e. Citronensäure oder Citronensäureanhydrat oder Natriumcitratanhydrat, oder ein geeignet proportioniertes Gemisch davon zu 5 bis 60 Gew.-%,
f. Süßungsmittel zu 0,1 bis 2 Gew.-%,
g. Aromastoff zu 0,1 bis 5 Gew.-%.

16. Verfahren zur Herstellung einer pharmazeutischen Zusammensetzung nach einem der vorangehenden Ansprüche, umfassend die folgenden Schritte
a. Mischen von Vildagliptin oder einem pharmazeutisch verträglichen Salz davon mit einem Sprengmittel, einem Hydrogencarbonat, einer Säure und Lactose oder mikrokristalliner Cellulose,
b. Hinzufügen eines Süßungsmittels und eines Aromastoffs in das vorstehend erzeugte Pulvergemisch und Mischen des sich ergebenden Gemisches,
c. Hinzufügen eines Fließregulierungsmittels und eines Gleitmittels in das vorstehend erhaltene Gemisch und Mischen des sich ergebenden Gemisches, und Abfüllen des resultierenden Pulvergemisches in Verpackungen in einer Portionsbeutelabfüllanlage.

17. Pharmazeutische Zusammensetzung nach einem der vorangehenden Ansprüche zur Vorbeugung oder Behandlung von Diabetes mellitus bei Säugetieren und insbesondere beim Menschen.

18. Pharmazeutische Zusammensetzung nach einem der vorangehenden Ansprüche, wobei die Zusammensetzung in Form eines Portionsbeutels vorliegt.

## Revendications

1. - Composition effervescente, administrée par voie orale après sa dispersion dans l'eau, **caractérisée par le fait qu'**elle comprend :
des particules contenant de la vildagliptine ou un sel pharmaceutiquement acceptable de vildagliptine, un acide et un carbonate, et
un emballage formant barrière à l'air et à l'humidité qui est rempli par lesdites particules.

2. - Composition pharmaceutique effervescente selon la revendication 1, dans laquelle ledit carbonate est un sel carbonate ou bicarbonate de métaux alcalins.

3. - Composition pharmaceutique selon l'une quelconque des revendications précédentes, dans laquelle ledit carbonate est choisi parmi le carbonate de sodium, le bicarbonate de sodium, l'anhydrate de bicarbonate de sodium, l'hydrogéno carbonate de sodium, le carbonate de calcium, le carbonate de sodium et de glycine ou parmi leurs mélanges en bonnes proportions.

4. - Composition pharmaceutique selon l'une quelconque des revendications précédentes, dans laquelle ledit acide est choisi parmi le citrate de sodium, l'acide citrique, l'acide tartrique, l'acide fumarique et l'acide malique ou parmi leurs mélanges en bonnes proportions.

5. - Composition pharmaceutique selon l'une quelconque des revendications précédentes, comprenant en outre au moins un ou plusieurs excipients.

6. - Composition pharmaceutique selon l'une quelconque des revendications précédentes, dans laquelle ledit excipient comprend au moins un ou un mélange en bonnes proportions de diluants, désintégrants, agents de glissement, lubrifiants, agents aromatisants et édulcorants.

7. - Composition pharmaceutique selon l'une quelconque des revendications précédentes, dans laquelle la dimension moyenne de particule (d50) de ladite composition se situe dans la plage de 250 à 600 µm.

8. - Composition pharmaceutique selon l'une quelconque des revendications précédentes, dans laquelle ledit diluant est le lactose, la cellulose microcristalline, un sucre ou un mélange en bonnes proportions de ceux-ci.

9. - Composition pharmaceutique selon l'une quelconque des revendications précédentes, dans laquelle ledit désintégrant est au moins un ou un mélange en bonnes proportions de polyvinylpyrrolidone et de glycolate d'amidon sodique.

10. - Composition pharmaceutique selon l'une quelconque des revendications précédentes, dans laquelle ledit agent de glissement est le dioxyde de silicium colloïdal.

11. - Composition pharmaceutique selon l'une quelconque des revendications précédentes, dans laquelle ledit lubrifiant comprend de préférence au moins un ou un mélange en bonnes proportions de polyéthylène glycol et de stéarate de magnésium.

12. - Composition pharmaceutique selon l'une quelconque des revendications précédentes, dans laquelle lesdits édulcorants comprennent au moins un ou un mélange en bonnes proportions d'aspartame, de sucralose, de saccharine ; de glucose, de lactose, de fructose et autres sucres, et de mannitol, de sorbitol, de xylitol, d'érythritol et autres alcools de sucres.

13. - Composition pharmaceutique selon l'une quelconque des revendications précédentes, dans laquelle lesdits agents aromatisants comprennent au moins un ou un mélange en bonnes proportions de menthol, de menthe poivrée, de cannelle, de chocolat, de vanilline et d'extraits de fruits tels que cerise, orange, fraise, raisin.

14. - Composition pharmaceutique selon l'une quelconque des revendications précédentes, comprenant les ingrédients suivants seulement :
a. vildagliptine ou un sel pharmaceutiquement acceptable de celle-ci à raison de 3,5 à 90 % en poids ;
b. lactose ou cellulose microcristalline ou un mélange en bonnes proportions de ceux-ci à raison de 5 à 90 % en poids ;
c. polyvinylpyrrolidone à raison de 1 à 30 % en poids ;
d. sels carbonates ou bicarbonates de métaux alcalins à raison de 5 à 60 % en poids ;
e. acide tartrique ou acide malique ou un mélange en bonnes proportions de ceux-ci à raison de 5 à 60 % en poids ;
f. édulcorant à raison de 0,1 à 2 % en poids ;
g. agent aromatisant à raison de 0,1 à 5 % en poids.

15. - Composition pharmaceutique selon l'une quelconque des revendications précédentes, comprenant les ingrédients suivants seulement :
a. vildagliptine ou un sel pharmaceutiquement acceptable de celle-ci à raison de 3,5 à 90 % en poids ;
b. lactose ou cellulose microcristalline ou un mélange en bonnes proportions de ceux-ci à raison de 5 à 90 % en poids ;
c. polyvinylpyrrolidone à raison de 1 à 30 % en poids ;
d. sels carbonates ou bicarbonates de métaux alcalins à raison de 5 à 60 % en poids ;
e. acide citrique ou anhydrate d'acide citrique ou anhydrate de citrate de sodium ou un mélange en bonnes proportions de ceux-ci à raison de 5 à 60 % en poids ;
f. édulcorant à raison de 0,1 à 2 % en poids ;
g. agent aromatisant à raison de 0,1 à 5 % en poids.

16. - Procédé de préparation d'une composition pharmaceutique selon l'une quelconque des revendications précédentes, comprenant les étapes de :
a. mélange de vildagliptine ou d'un sel pharmaceutiquement acceptable de celle-ci avec un désintégrant, un bicarbonate, un acide et du lactose ou de la cellulose microcristalline ;
b. addition d'un édulcorant et d'un agent aromatisant dans le mélange pulvérulent obtenu ci-dessus et mélange du mélange résultant ;
c. addition d'un agent de glissement et de lubrifiant dans le mélange obtenu ci-dessus et mélange du mélange résultant, et remplissage par le mélange pulvérulent résultant d'emballage dans une machine de remplissage de sachets.

17. - Composition pharmaceutique selon l'une quelconque des revendications précédentes pour prévenir ou traiter le diabète sucré chez les mammifères et en particulier les êtres humains.

18. - Composition pharmaceutique selon l'une quelconque des revendications précédentes, dans laquelle ladite composition se présente sous la forme d'un sachet.
